# EUROPEAN PATENT SPECIFICATION

(11) **EP 0 460 767 B1**
(45) Date of publication and mention of the grant of the patent: **10.01.1996**
(21) Application number: 91201401.6
(22) Date of filing: 06.06.1991
(51) Int. Cl.: C12M 1/00

(54) **A process and device for anaerobic fermentation**
Verfahren und Anlage zur anaeroben Gärung
Procédé et appareil pour la fermentation anaérobique

(30) Priority: 07.06.1990 NL 9001282
(43) Date of publication of application: 11.12.1991
(73) Proprietor: DUTCH ENVIRONMENTAL MANAGEMENT GROUP B.V., NL-5222 AV 's-Hertogenbosch (NL)
(72) Inventor: Masselink, Paul Herman Andreas Maria, NL-5235 BV 's-Hertogenbosch (NL)
(74) Representative: Kupecz, Arpad

(56) References cited:
- WO-A-82/02059
- FR-A- 327 974
- FR-A- 893 767
- FR-A- 1 006 958
- FR-A- 2 346 293
- GB-A- 2 041 403
- US-A- 4 204 842
- FSTA-JOURNAL; A. GRASSHOFF et al., no. 79-02-P0256/

## Description

The present invention relates to a process for the essentially anaerobic fermentation of organic material, for example originating from domestic waste or vegetable waste, as well as to a device for implementation of said process.

(A) WO-A-82/02059 discloses a process for anaerobic fermentation of organic waste which is converted into slurry in a mixing tank. After crushing said slurry is pumped into a closed holding container and then pumped via a heat exchanger into fermentation vessels.

Such a process is further known from, amongst others, the European patent application 0 131 319. In the described process organic materials are anaerobically fermented in a (semi-) continuous process, wherein during the feed of the device with fresh, fermentable material a part of the partially fermented material is removed from the reaction vessel, it is mixed with new organic material and it is recycled into said reaction vessel. Said organic material may originate from waste, from which heavy components, such as metals, glass etc. are removed, which organic material, if desired, may be reduced and homogenized.

A disadvantage of such a (semi-) continuous process is that in one single reaction vessel all stages of the fermentation process occur simultaneously, whereas each stage has its specific and optimum conditions. It should be clear that by the use of such a (semi-) continuous process in only one single fermentation vessel in favour of the occurrence of all stages of the fermentation process in said vessel the optimum conditions may not be met simultaneously.

An additional disadvantage of such a (semi-) continuous process is the requirement of a technical facility in the reaction vessel, with which material present on the bottom of the reaction vessel may be removed by means of a discharge canal, and which is permanently in contact with the material to be fermented. This gives rise to additional wear and maintenance. Furthermore, with the process described in the above-mentioned European patent application, a large portion of the (partially) fermented material is once again introduced into the fermentation vessel, which results in unnecessary circulation of not completely fermented organic material, which is disadvantageous for the economy of the process.

Moreover additional wear of the fermentation vessel and its components occurs by the repeated feed of mass on top and removal of mass at the bottom of the fermentation vessel.

Accordingly the object of the present invention is to provide a process and a device which effectively eliminate these disadvantages and allow batchwise, anaerobic fermentation of organic material.

This goal is now achieved by the present invention.

Therefore the invention provides a process of the kind described in the preamble, characterized in that said organic material is fed into a rotating drum-type container and there thoroughly mixed with a solid and/or liquid graft material containing anaerobic bacteria, after which the such obtained mass having a moisture content of 40 to 80%, by weight, is transferred into one of the fermentation compartments of an anaerobic fermentation installation comprising several rectangular fermentation compartments adjacent to each other in which said mass is heated and it is kept at the required temperature until the organic material is almost completely fermented while the biogas obtained from said fermentation is removed and whereafter the such obtained fermented material is removed entirely from said fermentation compartment.

An advantage of the present process is that in one single fermentation compartment organic material is present which occurs in one single fermentation stage of the anaerobic fermentation, resulting in almost ideal process conditions for the said stage in said fermentation compartment. All stages of the fermentation process are completed in succession in this compartment.

A further advantage is that the thus obtained, after fermentation to be removed material, is a homogeneously fermented mass, which improves the quality of the final product considerably.

Excess circulation of organic material is also avoided with the present process of batchwise fermentation which prevents unnecessary wear of parts of the device.

Another feature of the process of the invention is that prior to the anaerobic fermentation in a fermentation compartment of the fermentation device, the organic material is treated and simultaneously aereted in a rotating drum or tubular container which is preferably a so-called DANO drum (DANO is a registered trademark).

The effect of said aeration is, that due to aerobic decomposition processes the temperature of the organic material to be processed increases but does not achieve yet the optimum temperature that is required for the anaerobic fermentation, to wit about 55°C.

It is in particular advantageous with the present process when the organic material in said drum which is preferably a so-called DANO (registered trademark) drum is aerated prior to mixing it in the mixer and homogenizing the same. It should be noted that the aeration of the organic material also may take place in the storage.

Aeration of the organic materials in the storage happens specially if the waste to be processed mainly consists of vegetable material, for example originating from market withdrawn horticultural products. Accordingly, the organic waste materials which have to be processed, are, in addition to increasing the temperature by natural oxidation, usually heated in a rotating drum (which is preferably a DANO (registered trademark) drum) by injection of steam.

A further characteristic of the process according to the invention is that mixing occurs under aerobic conditions. In this variant the processable material, particularly domestic waste, is introduced in a container together with the graft material containing anaerobic bacteria, wherein said mass is thoroughly mixed. Usually, one uses for this purpose a rotating drum, preferably the so-called DANO (registered trademark) drum which is a rotating horizontally mounted tubular container. Such a tubular container is in open contact with the air. Prior to transport the thus obtained homogenized mass into a fermentation compartment of a fermentation device it is necessary to remove coarse non-fermertable components from the mass for example by means of sieves and magnets.

In said tubular container, which is preferably the so-called DANO (registered trademark) drum, said organic materials are squashed and reduced in order to improve the subsequent anaerobic degradation significantly.

An advantageous variant of the process of the invention is when during the aerobic treatment additional air is introduced resulting in a further rise of the temperature of the material to be fermented.

Another variant of the present process is characterized in that after the transfer of the obtained thoroughly mixed mass into one of the fermentation compartments said mass is heated to 30-60°C, preferably to 50-65°C, which temperature is maintained for the anaerobic fermentation of said mass.

It is noted that the required heat can be partly obtained by aeration whereas the optimum required heat preferably may be obtained by injection of steam in the rotating drum which is preferably a DANO (registered trademark) drum.

By doing so it is possible to bring the temperature of the mass to be fermented prior to its transfer into the fermentation compartment at a temperature required for the anaerobic fermentation, to wit 30-65°C.

According to another feature of the invention said graft material is heated prior to mixing it with said organic material to a temperature of 30-65°C, and preferably to 50-65°C.

By doing so the material to be fermented keeps the desired temperature after introduction of said graft material.

Furthermore, it is possible according to the present invention that said organic material optionally together with said graft material is heated to 30-65°C, and preferably to 50-65°C, and preferably by injection of steam.

As anaerobic graft material preferably use is made of fermented sewage sludge, fermented solid organic material or press liquid from dehydrators for fermented solid organic material or to this end specially grown biomass.

By using the fermented organic material or press liquid such graft material may be recovered from the fermentation compartment of the present anaerobic fermentation installation, so that there is no need to purchase and transport this graft material, which improves the economy of the process.

For a favourable working of the process of the invention said graft material is used in such an amount that excessive acidification of the mass in the particular fermentation compartment is prevented. Acidifying bacteria may not be present in excess. The medium should be such that the methane forming bacteria can exist. Excessive acifidication inhibits or even prevents proper fermentation.

An advantageous aspect of the proces of the invention is that the moisture content of the material to be fermented, introduced into a fermentation compartment, ranges from 40 to 80%, by weight.

This allows processing of various organic waste materials with a relatively high dry weight content.

A practical feature of the present process is the fact that the transport of graft material and material to be fermented to the particular fermentation compartment is effected with a conveying system.

The advantage of such a conveying system is that it permits the transport of material to be fermented with a relatively high dry weight content.

A further preferred aspect of the present process is that said transport is carried out by means of a closed conveying system, preferably a closed conveyor belt.

This prevents unnecessary heat loss of for example said thoroughly mixed mass and simultaneously the spreading of foul smells. For the transport use may be made of jack screw transport and prop transport means which are used for transport of wet sand. Preferably use is made of a traditional closed chain conveyor system or more preferably a closed conveyor belt.

Another feature of the present process concerns the fact that per fermentation compartment an amount of organic material to be fermented is introduced which corresponds to the average daily supply of new processable organic material.

In this respect it is favourable that in one fermentation compartment only organic material is present which is in the same fermentation stage, which organic material is kept there without interruption during the essentially entire period of the fermentation. This period varies depending on the process conditions from 15 to 50 days.

According to a variant of the process according to the invention the biogas formed during the fermentation is blown through the material to be fermented in a fermentation compartment. This results in a loosening up of the fermentable mass and in promoting the release of biogas.

Usually, at the end of the fermentation the fermented material in the particular fermentation compartment is blown through with an inert gas or air.

The advantage of this is that as much as possible biogas is obtained, because the obtained biogas is completely removed from the mass.

Finally, is it possible according to the present invention that the fermented material is treated aerobically before its removal from the fermentation compartment or after its removal. By doing so the not completely degraded material is aerobically processed and stabilized, which improves the quality of the obtained fermented final product.

The invention also comprises a device for carrying out the process of the invention. To this end the device is characterized in that said device is provided with at least a container for solid graft material and a container for liquid graft material, as well as a rotating drum-type container for thoroughly mixing of the organic material to be fermented and the solid and/or liquid graft material and which device further is provided with a conveying system for transport of the thoroughly mixed mass having a moisture content of 40 to 80%, by weight, from said drum-type container to one of the anaerobic rectangular fermentation compartments adjacent to each other of an anaerobic fermentation installation comprising several heatable fermentation compartments.

In such a device the mixing, usually under aerobic conditions, of the material to be fermented and the graft material may be carried out.

According to the invention each of the separate fermentation compartments may be provided with at one or more segmentation walls, if necessary, which depends on the size and capacity of the fermentation installation. Favourable results are obtained when in the fermentation compartment the segmentation walls are placed in a way that the relative distance between each other and the walls of the compartment is about the same. Furthermore, it is preferred when the height of the segmentation wall amounts about 5-15% less than the height of the partition wall of the fermentation compartment.

Advantageously such a segmentation wall as well as the partition wall of the compartment are provided with one or more heating elements which preferably comprise hot water heating elements.

According to a favourable embodiment of the present invention the device is provided with at least one container for fresh, fermentable organic material, in which it can be subjected to an aerobic treatment.

This embodiment will especially be used in case of with relatively clean, virtually not-contaminated, vegetable waste. In such a case the container is a simple mixing container.

According to the preferred embodiment of the device of the invention each of the fermentation compartments is provided with concrete partition walls wherein preferably a heating element is built in. Said concrete walls do have the advantage of a very good installation resulting in the maintenance of an optimum thermophilic temperature during the fermentation. Both the rear wall and the roof construction of the compartments are preferably made from an isolating sandwich panel or they are also made from concrete, in which case this is provided with isolating material at the outside surface in order to achieve an optimum isolating construction. Preferably, each of the mentioned fermentation compartments is provided with heatable floors which also contributes to bringing the mass to be fermented to a higher temperature as well as to maintain the required fermentation temperature. As mentioned before the heating is preferably achieved by means of a hot water heating system.

According to another feature of the device of the invention the fermentation compartments are moisture and air tight in order to enable an optimum anaerobic fermentation.

It is preferred when the device of the invention is provided with a closed conveying system between said separate containers for transport of said organic mass in any form, and/or said solid and/or said liquid graft material. Generally, a closed conveyor belt is used.

Said closed conveying system enables the transport of the mass streams without unnecessary heat loss and/or the causing of a stench inconvenience to the employees and environment.

The invention will be further explained by means of the accompanying drawings 1 and 2, wherein
Fig. 1 is a diagrammatic representation of the preferred embodiment of the invention, and

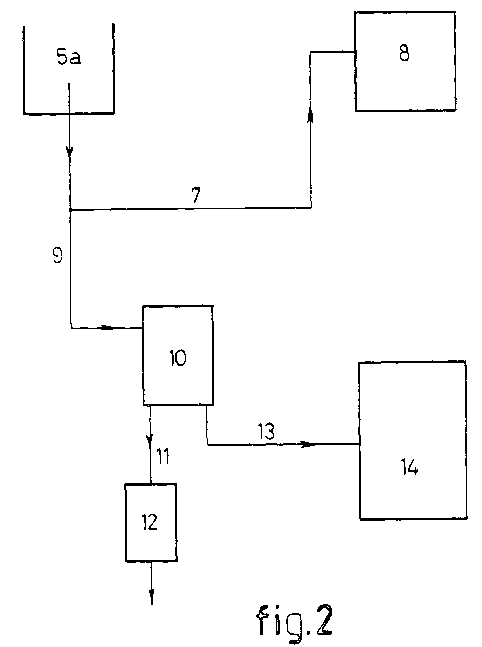

Fig. 2 provides a diagrammatic representation of an embodiment wherein the fermented material is subjected to a post-treatment.

### Figure 1

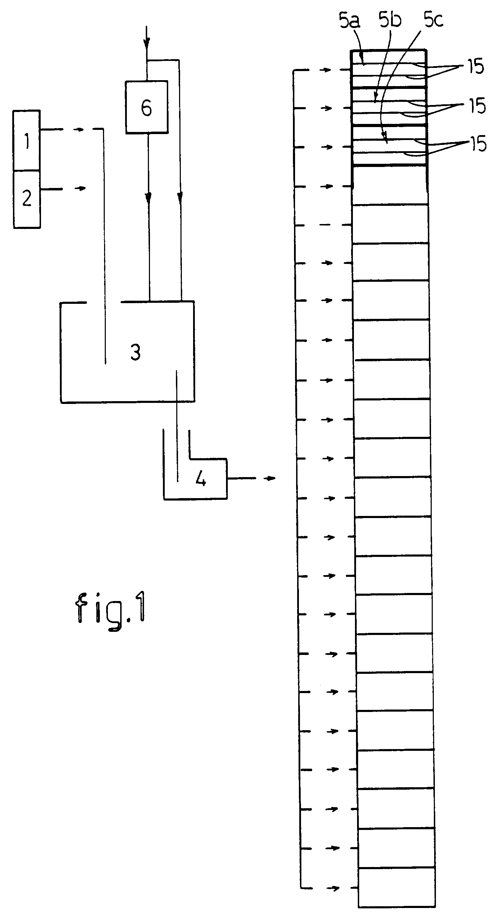

As mentioned before Fig. 1 represents the preferred embodiment of the device of the invention. Herein the material to be fermented is first, if required, pre-treated, in order to remove inert and/or interfering substances such as plastics and metals as much as possible. The said pre-treated organic material is generally transported into container 3 which is usually a rotating drum and preferably a so-called DANO (registered trademark) drum, wherein said material is reduced and thoroughly mixed. However, it is also possible to introduce the pre-treated material into a container 6 prior to its transportation into the container 3, wherein said organic material is subjected to aerobic degradation, which may be promoted by introducing air. To the organic material in a container 3 solid graft material from container 1 and liquid graft material from container 2 are added. However, it should be noted that said liquid graft material advantageously may be added to the organic material after leaving said container 3 and prior to its transportation into the fermentation compartiment 5a, 5b, 5c, ... by means of transportation installation 4. This option is not shown in Fig. 1. By doing so the anaerobic micro-organism content of the organic material to be fermented may easily be adjusted to the optimum level required for an optimum anaerobic fermentation in said fermentation compartment.

It should be clear that the mentioned pre-treatment of the organic material to be fermented is dependent on the origin of the waste material. In many cases the waste material is suitable for direct treatment in container 3, which is preferably a DANO (registered trademark) drum. Said DANO (registered trademark) drum is a horizontally arranged rotating tubular vessel, wherein the organic material to be fermented is mixed thoroughly in the presence of air. During this treatment aerobic degradation occurs, which causes the increase of the temperature of the moisture. However the temperature in said container 3 advantageously may be increased by injection of steam prior to its transfer to said fermentation compartment. The temperature of the organic mass is usually increased to 30-65°C, and preferably to 50-65°C. Between the container 3 and the transport installation 4 an additional container may be placed (not shown in the drawing) in order to obtain additional heating, grafting and water supply.

The size of a fermentation compartment, which compartment is preferably rectangular, for example 5a, depends on the average daily supply of new organic material to be fermented. Per working day one fermentation compartment will be filled, whereupon that compartment is closed off for the duration of the fermentation. The fermentation takes place during 15-50 days.

The production of the next day of material to be fermented is introduced into fermentation compartment 5b etc. In this way a batchwise charging of the fermentation installation is obtained.

It is noted that each of the fermentation compartments 5a, 5b, 5c, ... shown in the figure is provided with two segmentation walls 15 which preferably are provided with one or more heating elements (not shown in the figure). Said heating elements are advantageously hot water heating elements. However, the heating elements also may be the electrical elements. In case of hot water heating systems the required heat is provided by waste heat, which is developed by the gas engines, which convert the obtained biogas in electricity. Such fermentation compartments appear to ensure a good and efficient fermentation. However, it should be clear that the invention is not restricted to two segmentation walls per fermentation compartment. Dependant on the dimensions and therefore the capacity of the fermentation compartment one segmentation wall or three or more may be used, whereas in case of a small capacity no segmentation wall is used at all.

During operation of the fermentation installation essentially no organic material except biogas is withdrawn. Optionally press liquid which may be formed in the fermentation compartment may be recycled into the fermentation compartment in order to keep the water content of the organic mass at the required level.

The capacity of the fermentation compartments may be for example about 250 to 750m³. It will be clear that the smaller or larger fermentation compartments can be used. Usually the stacking height after charging of the material in the fermentation compartments is about 4.5 to 5.5 meters, whereas the capacity of the fermentation compartment may be accommodated by adapting the length of the compartments.

However, the total capacity of the fermentation installation may be increased by increasing the number of fermentation compartments.

The fermentation compartments may be constructed of many different materials. However, an acid resistant concrete construction is preferred. Each of the fermentation compartments (5a, 5b, 5c ...) preferably comprises a concrete floor, concrete partition walls and concrete segmentation walls, if any, whereas the rear wall and roof are made from isolating sandwich panels or they also may be made from concrete in which case the outer walls and roof are provided with an isolating material at the outside.

During the fermentation of the organic material biogas is formed, which is then removed. After the fermentation has been completed the material which is remained in the particular fermentation compartment is optionally treated with an inert gas or air. Then the contents of the particular fermentation compartment is removed. The above mentioned treatment with an inert gas or air also can take place after removal of the fermented material from the particular fermentation compartment.

### Post-treatment of the fermented material

### Figure 2

The fermented material may be removed from the fermentation compartment by means of an excavator. The fermented material from a fermentation compartment (for instance 5b) may be transported partially through transportation means such as a conveyor belt etc. 7 to a storage container 8 and it may serve as graft material for new fermentable organic material. The fermented sewage sludge may be brought to or maintained at a temperature of about 40-65°C. This permits that preferably each batch of new, fermentable organic material has the optimum temperature during the pre-treatment stage. It is also possible to transport the fermented material from a fermentation compartment, for example 5a, via transportation means 9 to a dehydrator 10, in which solid fermented organic material and press liquid from the dehydrator are transported by means of transport means 11 to a storage container 12 and by means of transportation means 13 to a storage container 14, respectively.

The solid organic material from storage container 12 may subsequently be dried and/or carried off.

The press liquid from storage container 14 and the fermented solid organic material from storage container 12 may be re-used as graft material for new, fermentable material.

The amount of graft material should always be sufficient to suppress the predominance of acid forming bacteria in the first stage of the fermentation process.

Once sufficient fermented material is obtained in the installation, which comprises several fermentation compartments, it is possible to use 30% of this fermented material as graft material in order to properly graft the new fermentable organic mass.

By the simultaneous use of fermented sewage sludge or press liquid from the dehydration of fermented organic material the percentage solid graft material may be reduced.

## Claims

1. A process for the essentially anaerobic fermentation of organic material, which originates for example from domestic waste or vegetable waste, **characterized** in that said organic material is fed into a rotating drum-type container and there thoroughly mixed with a solid and/or liquid graft material containing anaerobic bacteria, after which the such obtained mass having a moisture content of 40 to 80%, by weight, is transferred into one of the fermentation compartments of an anaerobic fermentation installation comprising several rectangular fermentation compartments adjacent to each other in which said mass is heated and it is kept at the required temperature until the organic material is almost completely fermented while the biogas obtained from said fermentation is removed and whereafter the such obtained fermented material is removed entirely from said fermentation compartment.

2. The process according to claim 1, **characterized** in that said organic material is aerated prior to the anaerobic fermentation.

3. The process according to claim 2, **characterized** in that said organic material is aerated prior to the mixing.

4. The process according to claim 1 or 2, **characterized** in that the mixing is carried out under aerobic conditions.

5. The process according to any of the claims 2 to 4, **characterized** in that during said aerobic treatment additional air is supplied.

6. The process according to any of the claims 1 to 5, **characterized** in that after transfer of the obtained thoroughly mixed mass into one of the fermentation compartments, said mass is heated to 30 to 65°C, which temperature is maintained for the anaerobic fermentation of said mass.

7. The process according to claim 6, **characterized** in that said mass is heated to 50-65°C, which temperature is maintained.

8. The process according to any of the claims 1 to 7, **characterized** in that said graft material is heated prior to mixing it with said organic material.

9. The process according to claim 8, **characterized** in that said graft material is heated to a temperature of 30-65°C, and usually to 50-65°C.

10. The process according to any of the claims 1 to 7, **characterized** in that said organic material together with said graft material is heated to 30-65°C, and usually to 50-65°C, generally by injection of steam.

11. The process according to any of the claims 1 to 10, **characterized** by the use of fermented sewage sludge, fermented solid organic material or press liquid from dehydrators for fermented solid organic material or to this end specially grown biomass as anaerobic graft material.

12. The process according to any of the claims 1 to 11, **characterized** in that the graft material is used in such an amount that excessive acidification of the mass in the particular fermentation compartment is prevented.

13. The process according to claim 1, **characterized** in that said moisture content of the material to be fermented is from 50 to 70%, by weight.

14. The process according to any of the claims 1 to 13, **characterized** in that the transport of graft material and material to be fermented to the particular fermentation compartment is effected with a conveying system.

15. The process according to claim 14, **characterized** in that said transport is carried out by means of a closed conveying system, such as a closed conveyor belt.

16. The process according to any of the claims 1 to 15, **characterized** in that per fermentation compartment an amount of organic material to be fermented is introduced which corresponds to the average daily supply of new, processable organic material.

17. The process according to any of the claims 1 to 16, **characterized** in that biogas formed during the fermentation is blown through the fermentable material in a fermentation compartment.

18. The process according to any of the claims 1 to 17, **characterized** in that at the end of the fermentation the fermented material in the particular fermentation compartment is blown through with an inert gas or air.

19. The process according to any of the claims 1 to 18, **characterized** in that the fermented material is treated aerobically before its removal from the fermentation compartment.

20. A device for the essentially anaerobic fermentation of organic material **characterized** in that said device is provided with at least a container (1) for solid graft material and a container (2) for liquid graft material, as well as a rotating drum-type container (3) for thoroughly mixing of the organic material to be fermented and the solid and/or liquid graft material and which device further is provided with a conveying system (4) for transport of the thoroughly mixed mass having a moisture content of 40 to 80%, by weight, from said drum-type container (3) to one of the anaerobic rectangular fermentation compartments adjacent to each other of an anaerobic fermentation installation (5) comprising several heatable fermentation compartments (5a, 5b, 5c, ...).

21. The device according to claim 20, **characterized** in that each of the separate fermentation compartments (5a, 5b, 5c, ...) is provided with one or more segmentation walls (15).

22. The device according to claim 21, **characterized** in that segmentation wall (15) and the partition walls are provided with one or more heating elements.

23. The device according to claim 22, **characterized** in that said heating elements are hot water heating elements.

24. The device according to claims 20 to 23, **characterized** in that it is provided with at least one container (6) for fresh, fermentable organic material, in which it can be subjected to an aerobic treatment.

25. The device according to any of the claims 20 to 24, **characterized** in that each of the fermentation compartments (5a, 5b, 5c, ...) comprises a concrete floor and concrete partition walls whereas the rear wall and roof are made from isolated sandwich panels or also from concrete, wherein the outer walls and roof are provided with an isolation material.

26. The device according to any of the claims 20 to 25, **characterized** in that each of the fermentation compartments (5a, 5b, 5c, ...) comprises a heatable bottom, if desired.

27. The device according to any of the claims 20 to 26, **characterized** in that the fermentation compartments (5a, 5b, 5c, ...) are moisture and air tight fermentation compartments.

28. The device according to any of the claims 20 to 27, **characterized** in that said device is provided with a closed conveying system between said separate containers for transport of said organic mass in any form, and/or said solid and/or said liquid graft material.

## Patentansprüche

1. Verfahren zur im wesentlichen anaeroben Gä_rung von organischem Material, das z. B. aus Hausabfall oder pflanzlichem Abfall entsteht, **dadurch gekennzeichnet**, daß das organische Material einem rotierenden trommelartigen Behälter zugeführt wird und dort eingehend mit einem festen und/oder flüssigen anaerobe Bakterien aufweisenden Graftmaterial gemischt wird, daß anschließend die so erhaltene Masse, die einen Feuchtigkeitsgehalt von 40 bis 80 Gewichts-% hat, in eines der Gärungsabteile einer anaeroben Gärungseinrichtung eingebracht wird, die mehrere einander benachbarte Gärungsabteile aufweist, in denen die Masse erhitzt wird und in denen sie bei der erforderlichen Temperatur solange verbleibt, bis das organische Material nahezu vollständig vergärt ist, während das durch die Gärung erhaltene Biogas abgezogen wird und wonach das so erhaltene vergärte Material aus dem Gärungsabteil vollständig entfernt wird.

2. Verfahren nach Anspruch 1, **dadurch gekennzeichnet**, daß das organische Material vor der anaeroben Gärung belüftet wird.

3. Verfahren nach Anspruch 2, **dadurch gekennzeichnet**, daß das organische Material vor dem Mischen belüftet wird.

4. Verfahren nach Anspruch 1 oder 2, **dadurch gekennzeichnet**, daß das Mischen unter aeroben Bedingungen durchgeführt wird.

5. Verfahren nach einem der Ansprüche 2 bis 4, **dadurch gekennzeichnet**, daß während der aeroben Behandlung zusätzliche Luft zugeführt wird.

6. Verfahren nach einem der Ansprüche 1 bis 5, **dadurch gekennzeichnet**, daß nach Überführung der erhaltenen, eingehend gemischten Masse in eines der Gärungsabteile die Masse auf 30 bis 65° C erhitzt wird, wobei die Temperatur für die anaeroben Gärung der Masse aufrecht erhalten bleibt.

7. Verfahren nach Anspruch 6, **dadurch gekennzeichnet**, daß die Masse auf 50 bis 65° C erhitzt wird, wobei die Temperatur aufrecht erhalten bleibt.

8. Verfahren nach einem der Ansprüche 1 bis 7, **dadurch gekennzeichnet**, daß das Graftmaterial vor dem Mischen mit dem organischen Material erhitzt wird.

9. Verfahren nach Anspruch 8, **dadurch gekennzeichnet**, daß das Graftmaterial auf eine Temperatur von 30 bis 65° C und gewöhnlich auf 50 bis 65° C erwärmt wird.

10. Verfahren nach einem der Ansprüche 1 bis 7, **dadurch gekennzeichnet**, daß das organische Material mit dem Graftmaterial auf 30 bis 65° C und üblicherweise auf 50 bis 65° C erwärmt wird, im allgemeinen durch Injektion von Dampf.

11. Verfahren nach einem der Ansprüche 1 bis 10, **gekennzeichnet durch** die Verwendung von vergärtem Abwasserschlamm, vergärtem festen organischen Material oder Pressflüssigkeit von Entwässerungsmitteln für vergärtes festes organisches Material oder speziell zu diesem Zweck gewachsene bzw. angebaute Biomasse als anaerobes Graftmaterial.

12. Verfahren nach einem der Ansprüche 1 bis 11, **dadurch gekennzeichnet**, daß das Graftmaterial in einer solchen Menge verwendet wird, daß eine übermässige Säuerung der Masse in dem betreffenden Gärungsabteil vermieden wird.

13. Verfahren nach Anspruch 1, **dadurch gekennzeichnet**, daß der Flüssigkeitsgehalt des zu vergärenden Materials zwischen 50 bis 70 Gewichts-% beträgt.

14. Verfahren nach einem der Ansprüche 1 bis 13, **dadurch gekennzeichnet**, daß der Transport des Graftmaterials und des zu vergärenden Materials zu dem betreffenden Gärungsabteil mit einem Fördersystem erfolgt.

15. Verfahren nach Anspruch 14, **dadurch gekennzeichnet**, daß der Transport über ein geschlossenes Fördersystem, wie beispielsweise einen geschlossenen Fördergurt durchgeführt wird.

16. Verfahren nach einem der Ansprüche 1 bis 15, **dadurch gekennzeichnet**, daß pro Gärungsabteil eine solche Menge an organischem, zu vergärenden Material zugeführt wird, die der durchschnittlichen täglichen Lieferung von neuem zu verarbeitendem organischen Material entspricht.

17. Verfahren nach einem der Ansprüche 1 bis 16, **dadurch gekennzeichnet**, daß das während der Gärung gebildete Biogas durch das zu vergärende Material in einem Gärungsabteil geblasen wird.

18. Verfahren nach einem der Ansprüche 1 bis 17, **dadurch gekennzeichnet**, daß das vergärte Material am Ende der Gärung in dem betreffenden Gärungsabteil mit einem Inertgas oder Luft durchgeblasen wird.

19. Verfahren nach einem der Ansprüche 1 bis 18, **dadurch gekennzeichnet**, daß das vergärte Material vor seiner Entnahme aus dem Gärungsabteil aerob behandelt wird.

20. Vorrichtung zur im wesentlichen anaeroben Gärung von organischem Material, **dadurch gekennzeichnet**, daß die Vorrichtung mit wenigstens einem Behälter (1) für festes Graftmaterial und einem Behälter (2) für flüssiges Graftmaterial versehen ist, sowie mit einem rotierenden trommelartigen Behälter (3) zum eingehenden Mischen des organischen, zu gärenden Materials und des festen und/oder flüssigen Graftmaterials, und wobei die Vorrichtung weiterhin versehen ist mit einem Fördersystem (4) zum Transport der eingehend gemischten, einen Feuchtigkeitsgehalt von 40 bis 80 Gewichts-% aufweisenden Masse von dem trommelartigen Behälter (3) zu einem der anaeroben, rechteckigen, benachbart zueinander angeordneten Gärungsabteile einer anaeroben Gärungseinrichtung (5), die wiederum mehrere beheizbare Gärungsabteile (5a, 5b, 5c ) aufweist.

21. Vorrichtung nach Anspruch 20, **dadurch gekennzeichnet**, daß jedes der separaten Gärungsabteile (5a, 5b, 5c ) mit einer oder mehreren Segmentationswandungen (15) versehen ist.

22. Vorrichtung nach Anspruch 21, **dadurch gekennzeichnet**, daß die Segmentationswandung (15) und die Trennwandungen mit einem oder mehreren Heizelementen versehen sind.

23. Vorrichtung nach Anspruch 22, **dadurch gekennzeichnet**, daß die Heizelemente als Heißwasser-Heizelemente ausgebildet sind.

24. Vorrichtung nach Anspruch 20 bis 23, **dadurch gekennzeichnet**, daß wenigstens ein Behälter (6) für frisches, zu vergärendes organisches Material vorgesehen ist, in dem es einer aeroben Behandlung unterzogen werden kann.

25. Verfahren nach einem der Ansprüche 20 bis 24, **dadurch gekennzeichnet**, daß jedes der Gärungsabteile (5a, 5b, 5c ) einen Betonboden und Betontrennwände aufweist, während die Rückwandung und die Decke aus isolierten Verbundplatten oder auch aus Beton bestehen, wobei die äußeren Wandungen und die Decke mit einem isolierten Material versehen sind.

26. Vorrichtung nach einem der Ansprüche 20 bis 25, **dadurch gekennzeichnet**, daß jedes der Gärungsabteile (5a, 5b, 5c ) einen beheizbaren Boden aufweist, soweit dies erwünscht ist.

27. Vorrichtung nach einem der Ansprüche 20 bis 26, **dadurch gekennzeichnet**, daß die Gärungsabteile (5a, 5b, 5c, ...) als flüssigkeits- und luftdichte Gärungsabteile ausgebildet sind.

28. Vorrichtung nach einem der Ansprüche 20 bis 27, **dadurch gekennzeichnet**, daß die Vorrichtung versehen ist mit einem geschlossenen Fördersystem zwischen den einzelnen Behältern zum Transport der organischen Masse in jeder Form und/oder des festen und/oder flüssigen Graftmaterials.

## Revendications

1. Procédé de fermentation pratiquement anaérobie d'une matière organique, qui a pour origine par exemple des déchets domestiques ou des déchets de légumes, caractérisé en ce que ladite matière organique est envoyée à l'intérieur d'un conteneur du type tambour rotatif et y est vigoureusement mélangée avec une matière porteuse de germes solide et/ou liquide contenant des bactéries anaérobies, après quoi la masse ainsi obtenue, ayant une teneur en humidité de 40 à 80% en poids, est transférée à l'intérieur de l'un des compartiments de fermentation d'une installation de fermentation anaérobie comportant plusieurs compartiments rectangulaires de fermentation adjacents les uns aux autres dans lequel ladite masse est chauffée et est maintenue au niveau de la température requise jusqu'à ce que la matière organique soit pratiquement entièrement fermentée alors que les biogaz obtenus à partir de ladite fermentation sont enlevés et, après ceci, la matière fermentée ainsi obtenue est entièrement enlevée dudit compartiment de fermentation.

2. Procédé selon la revendication 1, caractérisé en ce que ladite matière organique est aérée avant la fermentation anaérobie.

3. Procédé selon la revendication 2, caractérisé en ce que ladite matière organique est aérée avant le mélange.

4. Procédé selon la revendication 1 ou 2, caractérisé en ce que le mélange est exécuté dans des conditions aérobies.

5. Procédé selon l'une quelconque des revendications 2 à 4, caractérisé en ce que pendant le traitement aérobie, de l'air supplémentaire est alimenté.

6. Procédé selon l'une quelconque des revendications 1 à 5, caractérisé en ce qu'après transfert de la masse vigoureusement mélangée obtenue jusqu'à l'intérieur de l'un des compartiments de fermentation, ladite masse est chauffée entre 30 et 65°C, laquelle température est maintenue pendant la fermentation anaérobie de ladite masse.

7. Procédé selon la revendication 6, caractérisé en ce que ladite masse est chauffée entre 50 et 65°C, laquelle température est maintenue.

8. Procédé selon l'une quelconque des revendications 1 à 7, caractérisé en ce que ladite matière porteuse de germes est chauffée avant son mélange avec ladite matière organique.

9. Procédé selon la revendication 8, caractérisé en ce que ladite matière porteuse de germes est chauffée à une température située entre 30 et 65°C, et habituellement entre 50 et 65°C.

10. Procédé selon l'une quelconque des revendications 1 à 7, caractérisé en ce que ladite matière organique associée à ladite matière porteuse de germes est chauffée entre 30 et 65°C, et habituellement entre 50 et 65°C, de manière générale par injection de vapeur.

11. Procédé selon l'une quelconque des revendications 1 à 10, caractérisé en ce qu'on utilise en tant que matière porteuse de germes anaérobie de la boue d'eau d'égout fermentée, une matière organique solide fermentée, ou du liquide de pressage provenant de déshydrateurs de matière organique solide fermentée, ou à cet effet, une biomasse mise à croître de manière spéciale.

12. Procédé selon l'une quelconque des revendications 1 à 11, caractérisé en ce que la matière porteuse de germes est utilisée selon une quantité telle qu'on empêche l'acidification excessive de la masse dans le compartiment particulier de fermentation.

13. Procédé selon la revendication 1, caractérisé en ce que ladite teneur en humidité de la matière destinée à être fermentée est d'environ 50 à 70% en poids.

14. Procédé selon l'une quelconque des revendications 1 à 13, caractérisé en ce que le transport de matière porteuse de germes et de matière destinée à être fermentée vers le compartiment particulier de fermentation est effectué à l'aide d'un système de convoyage.

15. Procédé selon la revendication 14, caractérisé en ce que ledit transport est exécuté par l'intermédiaire d'un système de convoyage fermé, tel qu'une bande de convoyeur fermé.

16. Procédé selon l'une quelconque des revendications 1 à 15, caractérisé en ce que par compartiment de fermentation on introduit une quantité de matière organique destinée à être fermentée qui correspond à la moyenne d'alimentation quotidienne en nouvelle matière organique pouvant être traitée.

17. Procédé selon l'une quelconque des revendications 1 à 16, caractérisé en ce que les biogaz formés pendant la fermentation sont soufflés à travers la matière pouvant fermenter située dans un compartiment de fermentation.

18. Procédé selon l'une quelconque des revendications 1 à 17, caractérisé en ce qu'à la fin de la fermentation la matière fermentée dans le compartiment particulier de fermentation est traversée par soufflage d'un gaz inerte ou d'air.

19. Procédé selon l'une quelconque des revendications 1 à 18, caractérisé en ce que la matière fermentée est traitée de manière aérobie avant d'être enlevée du compartiment de fermentation.

20. Dispositif destiné à la fermentation pratiquement anaérobie d'une matière organique, caractérisé en ce que ledit dispositif est muni d'au moins un conteneur (1) destiné à une matière porteuse de germes solide et un conteneur (2) destiné à une matière porteuse de germes liquide, ainsi que d'un conteneur (3) du type tambour rotatif destiné à mélanger vigoureusement la matière organique destinée à être fermentée et la matière porteuse de germes solide et/ou liquide et le dispositif est muni en outre d'un système de convoyage (4) destiné au transport de la masse vigoureusement mélangée ayant une teneur en humidité de 40 à 80% en poids, depuis ledit conteneur (3) du type tambour vers l'un des compartiments rectangulaires de fermentation anaérobie adjacents les uns aux autres d'une installation de fermentation anaérobie (5) comportant plusieurs compartiments de fermentation pouvant être chauffés (5a, 5b, 5c, ...).

21. Dispositif selon la revendication 20, caractérisé en ce que chacun des compartiments de fermentation séparés (5a, 5b, 5c, ...) est muni d'une ou de plusieurs parois (15) de séparation en tronçons.

22. Dispositif selon la revendication 21, caractérisé en ce que la paroi (15) de séparation en tronçons et les parois de séparation sont munies d'un ou de plusieurs éléments de chauffage.

23. Dispositif selon la revendication 22, caractérisé en ce que lesdits éléments de chauffage sont des éléments de chauffage à eau chaude.

24. Dispositif selon l'une quelconque des revendications 20 à 23, caractérisé en ce qu'il comporte au moins un conteneur (6) pour de la matière organique fraîche pouvant être fermentée, dans lequel elle peut être soumise à un traitement aérobie.

25. Dispositif selon l'une quelconque des revendications 20 à 24, caractérisé en ce que chacun des compartiments de fermentation (5a, 5b, 5c, ...) est constitué d'un plancher en béton et de parois de séparation en béton alors que la paroi arrière et le toit sont constitués de panneaux isolés du type sandwich ou aussi de béton, les parois extérieures et le toit étant munis d'un matériau isolant.

26. Dispositif selon l'une quelconque des revendications 20 à 25, caractérisé en ce que chacun des compartiments de fermentation (5a, 5b, 5c, ...) est constitué d'un fond pouvant être chauffé si on le désire.

27. Dispositif selon l'une quelconque des revendications 20 à 26, caractérisé en ce que les compartiments de fermentation (5a, 5b, 5c, ...) sont des compartiments de fermentation étanches à l'humidité et à l'air.

28. Dispositif selon l'une quelconque des revendications 20 à 27, caractérisé en ce que ledit dispositif est muni d'un système de convoyage fermé entre lesdits conteneurs séparés pour transporter ladite masse organique sous une forme quelconque, et/ou ladite matière porteuse de germes solide et/ou ladite matière porteuse de germes liquide.
